# EUROPEAN PATENT SPECIFICATION

(11) **EP 2 961 255 B1**
(45) Date of publication and mention of the grant of the patent: **13.04.2022**
(21) Application number: 13876155.6
(22) Date of filing: 27.12.2013
(51) Int. Cl.: A61B 8/08, A61B 17/34

(54) **HANDHELD ULTRASOUND IMAGING DEVICE COMPRISING A MARKING UNIT**
HANDHALTBARE ULTRASCHALLBILDGEBUNGSVORRICHTUNG MIT MARKIERUNGSEINHEIT
DISPOSITIF D'IMAGERIE ULTRASONORE PORTATIF COMPRENANT UNE UNITÉ DE MARQUAGE

(30) Priority: 28.02.2013 US 201361770448 P
(43) Date of publication of application: 06.01.2016
(60) Divisional application: 20150694.6
(73) Proprietor: Rivanna Medical, Inc., Charlottesville, VA 22911 (US)
(72) Inventor: MAULDIN, Frank, William, Charlottesville, VA 22903 (US); OWEN, Kevin, Crozet, VA 22932 (US); MURRAY, Christopher, Richmond, VA 23219 (US); FERRIS, Bruce, Richmond, VA 23220 (US)
(74) Representative: Forresters IP LLP
(86) International application number: PCT/US2013/077917
(87) International publication number: WO 2014/133665

(56) References cited:
- WO-A1-01/13796
- WO-A1-2012/178109
- WO-A2-2010/019795
- US-A- 6 126 608
- US-A- 6 139 496
- US-A1- 2005 085 727
- US-A1- 2007 106 156
- US-A1- 2007 167 808
- US-A1- 2009 043 204
- US-A1- 2010 179 429
- US-A1- 2010 312 120
- US-A1- 2011 023 585
- US-A1- 2011 166 451
- US-A1- 2012 289 829
- US-A1- 2014 005 542

## Description

### TECHNICAL FIELD

Imaging devices for identification of target regions are generally described.

### BACKGROUND

Medical ultrasound is a popular medical imaging modality primarily used for diagnostic imaging of soft tissue but also for interventional procedures such as guidance of a needle or catheter placement. Examples include diagnostic imaging of organs, such cardiac or liver structures. Common interventional procedures that rely on ultrasound guidance are central line placement and guidance of nerve blocks, both of which are high volume procedures in certain hospital settings such as the intensive care unit (ICU). Current ultrasound systems are mostly cart-based and optimized for superior contrast and resolution in soft tissue. However, these systems are generally expensive, and in a hospital setting with multiple physicians per ultrasound system, they can be difficult to access.

US 6139486 A discloses a handheld ultrasound imaging device comprising a housing and an ultrasound imaging unit disposed within the housing. WO 2012/178109 A1 concerns a needle guide attachable to an ultrasound probe unit. WO 2010/019795 A2 discloses a needle guide for catheter delivery. From US 2007/167808 A1, WO01/13796 A1 and US2005/085727 A1 a detachable marking device for a ultrasound probe are known, wherein US 2005/085727 A1 discloses a device that can be readily attached to and removed from a diagnostic ultrasound transducer and is equipped with a marker element for indenting the skin of a patient. US 2010/312120 A1, US 2010/179429 A1, US 2011/166451 A1 and US 2012/289829 A1 concern a handheld ultrasound imaging device comprising a housing with a display, which is rotational about a single axis.

### SUMMARY

The invention is defined in the appended claims. Handheld ultrasound imaging devices are generally described. Certain embodiments have improved portability relative to prior imaging systems. In addition, attachable accessories that facilitate interventional procedures (including interventional ultrasound procedures) involving directing a probe (e.g., needle or catheter) to a probe target (e.g., blood vessel or nerve bundle) are also described. Certain embodiments can be used in handheld imaging devices, which address many of the limitations of prior art systems by, for example, providing standard imaging capabilities in a low cost portable device.

In accordance with the invention, a handheld ultrasound imaging device as defined in claim 1 is provided. The handheld ultrasound imaging device comprises a housing comprising a first end comprising an ultrasound imaging unit, a second end comprising a display, and a hand grip region between the ultrasound imaging unit and the display.

The handheld ultrasound imaging device further comprises a marking unit detachably coupled to the housing and comprising a probe indicator configured to indicate proper placement of a probe at or near a target that is to be imaged, and positioned such that, during use, a line extending between the target and the ultrasound imaging unit intersects the marking unit.

The handheld ultrasound imaging device comprises a housing comprising an ultrasound imaging unit, a hand grip region, and a rotatable display, wherein the rotatable display is configured to be rotated, relative to at least another portion of the housing, about at least one rotational axis by at least about 30 degrees.

The handheld ultrasound imaging device may comprise, according to some embodiments, an actuator on the hand grip region configured such that when the actuator is activated, data from the ultrasound imaging unit is recorded and/or manipulated. The actuator may be, according to certain embodiments, configured to perform one or more of a number of other functions including, but not limited to, video save, device power on/off, image settings adjustment (e.g., imaging mode, gain, frequency, contrast, depth), and/or menu navigation.

The handheld ultrasound imaging device is configured to produce an image along a scanning plane. In certain embodiments, the housing is elongated and comprises a longitudinal axis. In some embodiments, the smallest angle between the scanning plane of the imaging unit and the longitudinal axis of the housing is less than about 45°. In certain embodiments, the scanning plane of the imaging unit is substantially parallel to the longitudinal axis of the housing. According to certain embodiments, the rotatable display is configured to be rotated such that the display can be oriented in a first position substantially parallel to a scanning plane of the imaging unit and in a second position substantially perpendicular to the scanning plane of the imaging unit. In some embodiments, the handle is located directly above the imaging unit during operation of the imaging device.

In some embodiments, the imaging device occupies a volume of equal to or less than about 500 cm³.

A cover for the imaging device may be provided that comprises, in certain embodiments, a cover body configured to be attached to the imaging device .

The cover can have any one or more of the following properties, according to various embodiments. In some embodiments, the cover is configured such that when it is attached to the imaging device, an image can be produced without the use of transmission medium. For example, in certain embodiments, the cover is configured such that when it is attached to an ultrasound imaging device, an image can be produced without the use of ultrasound gel or other ultrasound transmission medium.

In some embodiments,
the marking unit comprises an adhesive material configured to adhere the marking unit to a surface of a target. In accordance with the invention, the marking unit comprises a moveable tab
configured to indicate a site corresponding to a center of a scan plane. The moveable tab is configured to make a visible indentation at a site
corresponding to the center of the scan plane. The probe can be, in certain embodiments,
a needle and/or a catheter. For example, according to certain embodiments, the marking unit attached to the cover body is configured to indicate proper placement of a needle or a catheter.

Other advantages and novel features of the present invention will become apparent from the following detailed description of various non-limiting embodiments of the invention when considered in conjunction with the accompanying figures.

### BRIEF DESCRIPTION OF THE DRAWINGS

Non-limiting embodiments of the present invention will be described by way of example with reference to the accompanying figures, which are schematic and are not intended to be drawn to scale. In the figures, each identical or nearly identical component illustrated is typically represented by a single numeral. For purposes of clarity, not every component is labeled in every figure, nor is every component of each embodiment of the invention shown where illustration is not necessary to allow those of ordinary skill in the art to understand the invention. In the figures:
FIG. 1 is a schematic illustration of an exemplary imaging device, according to certain embodiments, wherein the detachably coupled marking unit is detached and not shown;
FIGS. 2A-2C are, according to one set of embodiments, a series of schematic illustrations outlining the rotation of a display of an exemplary imaging device;
FIG. 3A is a schematic illustration of an imaging device, not independently claimed, comprising an exemplary detachable marking unit, according to some aspects;
FIG. 3B is a schematic cross-sectional illustration of a marking unit, not independently claimed, according to certain aspects;
FIGS. 4A-4D are a series of schematic illustrations showing the use of an exemplary detachable marking unit, not independently claimed, according to certain aspects;
FIGS. 5A-5E are, according to certain embodiments, a series of schematic illustrations showing the use of an exemplary detachable marking unit;
FIG. 6 is a series of schematic illustrations showing the connectivity between an exemplary detachable marking device and an imaging device, not independently claimed, according to some aspects;
FIG. 7 is a schematic illustration of an exemplary detachable marking unit comprising a needle sleeve, not independently claimed according to certain aspects; and
FIG. 8 is an exemplary imaging unit display, according to one set of embodiments.

### DETAILED DESCRIPTION

While the invention is as defined in the appended claims, imaging devices for identification of target regions are generally described.

Certain embodiments relate to handheld ultrasound imaging devices. Various embodiments are related to configurations that may provide, in some cases but not necessarily all cases, one or more advantages during operation of the imaging device.

Certain embodiments are related to handheld ultrasound imaging devices in which the imaging unit, the display, and the handgrip region are positioned relative to each other such that operation of the imaging device is made easier, under certain circumstances. In one set of embodiments, the handheld ultrasound imaging device comprises a housing, which can include an ultrasound imaging unit at one end, a display at another end, and a handgrip region between the ultrasound imaging unit and the display. As described in more detail below, arranging the imaging unit, the hand grip region, and the display in this manner can allow one to direct the movement of the handheld device relatively easily (e.g., by resting the side of the hand on a surface of the object that is being imaged) while maintaining the ability to see the display screen.

Certain embodiments are related to the use of rotatable displays in handheld ultrasound imaging devices. Generally, ultrasound imaging devices in the past have included fixed displays or displays that are spatially separated from the housing in which the imaging unit is contained. Imaging devices with fixed displays can be difficult to operate, as they do not allow one to adjust the angle of the display relative to the eye of the user without rotating or otherwise moving the housing to which the display is connected. While imaging devices with displays that are spatially separated from the device housing allow one to adjust the position of the display relative to the imaging unit, they are generally difficult to transport and/or store when they are not in use. By integrating the display with the housing while maintaining the ability to rotate the display relative to the housing one can realize the benefit of portability and ease of storage while also allowing for relatively easy repositioning of the display relative to the users eye.

Some embodiments are directed to configurations of marking units - configured to direct the placement of a probe such as a needle or catheter - in the handheld ultrasound imaging devices. For example, according to certain embodiments, a handheld ultrasound imaging device can comprise a marking unit detachably coupled to the housing of the device and positioned such that the target that is being imaged, the ultrasound imaging unit, and the marking unit are aligned during use. Maintaining alignment of these components can allow one, according to certain embodiments, to properly align the marking unit relative to the target relatively easily during use. As described in more detail below, this alignment, according to some embodiments, may allow one to position the marking unit while the imaging unit is still in place, which can, according to certain embodiments, improve the accuracy of the placement of a probe such as a needle, a catheter, or other device.

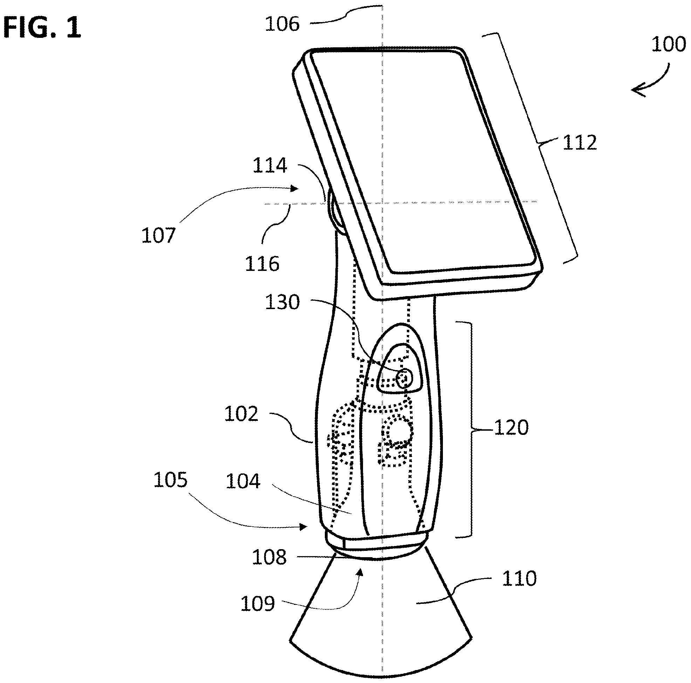

FIG. 1 is an exemplary schematic illustration of a handheld ultrasound imaging device 100, according to certain embodiments. In certain embodiments, the handheld ultrasound imaging device comprises a housing. For example, in FIG. 1, imaging device 100 comprises housing 102. According to certain embodiments, the housing comprises a first end associated with an ultrasound imaging unit. Referring to FIG. 1, for example, imaging unit 104 is associated with end 105 of housing 102. As shown in FIG. 1, ultrasound imaging unit 104 is enclosed within housing 102. Other arrangements are also possible.

In some embodiments, imaging unit 104 can comprise, for example, an ultrasound transducer 108. The ultrasound imaging unit 104 can be configured, in certain embodiments, to produce an image along at least one scanning plane 110. The imaging unit can be configured to produce an image using standard ultrasound image processing techniques known to those of ordinary skill in the art of ultrasound imaging, and described in more detail below.

The imaging device 100 comprises, in certain embodiments, display 112 *(e.g.,* an LCD display, an OLED display, or any other suitable type of display). The display can be located at a second end of the housing. For example, in FIG. 1, display 112 is located at end 107 of housing 102. According to certain embodiments, ultrasound transducer 108 can be configured to produce data along at least one scanning plane 110 that can be subsequently output on display 112.

In certain embodiments, an ultrasound transducer surface (e.g., configured to be applied on or near a target site) can be located at one end of the housing and the display can be attached to the housing at the opposite end. For example, in FIG. 1, ultrasound transducer surface 109 is located at end 105 of housing 102, and display 112 is located at second end 107 of housing 102. In certain embodiments, the display can be directly attached to the top of the housing. For example, in FIG. 1, display 112 is directly attached to the top of housing 102.

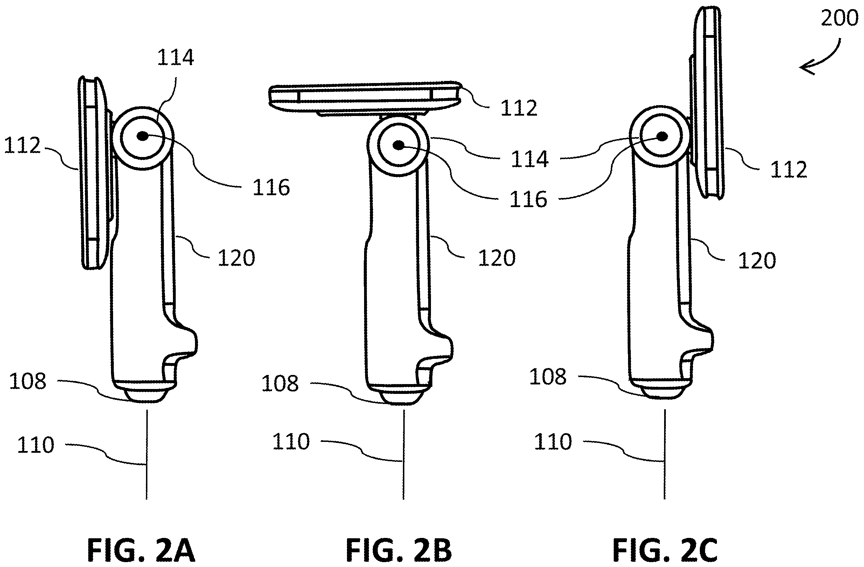

According to certain embodiments, the display of the imaging unit can be a rotatable display. For example, referring to FIG. 1, display 112 can be connected to housing 120 via pivot 114, which can allow rotatable display 112 to rotate about at least one rotational axis 116. Any suitable pivot may be used. For example, as shown in FIG. 1, cylindrical pivot 114 can be used to rotate display 112 about a single rotational axis 116. FIGS. 2A-2C are exemplary schematic illustrations showing the rotation of display 112 about single rotational axis 116 (going into and out of the page in FIGS. 2A-2C). In FIGS. 2A-2C, display 112 is rotated about device hand grip region 120. In certain embodiments, pivot 114 comprises a ball-and-socket arrangement or other type of arrangement, which can allow for rotation of display 112 about multiple rotational axes. In some such embodiments, display 112 can be rotated about more than one axis so that twisting or rotation in other dimensions is allowed. In other such embodiments, the display 112 can be attached to the housing via magnets, hook and loop fastener (e.g., Velcro), or other attachment means.

In some embodiments, the rotatable display is configured to be rotated, relative to at least another portion of the housing (e.g., the hand grip region, the imaging unit, and/or another portion of the housing), about at least one rotational axis by at least about 30 degrees, at least about 60 degrees, at least about 90 degrees, at least about 120 degrees, at least about 150 degrees, or at least about 175 degrees. For example, in FIGS. 2A-2C, display 112 is illustrated as being rotated about both hand grip region 120 and ultrasound transducer 108 by 180 degrees.

In certain embodiments, the rotatable display is configured to be rotated such that the display can be oriented in a first position substantially parallel to a scanning plane of the imaging unit and in a second position substantially perpendicular to the scanning plane of the imaging unit. For example, in FIGS. 2A and 2C, imaging device 100 is shown in which display 112 is substantially parallel to scanning plane 110 of imaging device 100. In addition, in FIG. 2B, display 112 is substantially perpendicular to scanning plane 110 of imaging device 100. Display 112 can be said to be in a 0-degree configuration in FIG. 2A, a 90-degree configuration in FIG. 2B, and a 180-degree configuration in FIG. 2C. In the exemplary embodiment of FIGS. 2A-2C, display 112 can be configured to a position at any angle relative to scan plane 110 of imaging device 100 within the range between the 0-degree configuration (FIG. 2A) and the 180-degree configuration (FIG. 2C).

While a rotatable display has been described in association with the above embodiments, it should be understood that in other embodiments, non-rotatable displays could be used.

In certain embodiments, the housing is elongated and comprises a longitudinal axis. For example, referring back to FIG. 1, housing 102 is elongated and comprises longitudinal axis 106.

In some embodiments, the imaging device is configured such that the smallest angle between the scanning plane 110 of the ultrasound imaging unit 104 and the longitudinal axis 106 of the device housing 102 is relatively small (e.g., less than about 45°, less than about 30°, less than about 15°, less than about 5°). In certain embodiments, the smallest angle between the scanning plane 110 of the ultrasound imaging unit 104 and the longitudinal axis 106 of the device housing 102 is less than about 1°, in which case, the scanning plane 110 of the ultrasound imaging unit 104 is said to be substantially parallel to the longitudinal axis 106 of the device housing 102.

In some embodiments, the imaging device can be relatively small. For example, in certain embodiments, the imaging device may occupy a volume of equal to or less than about 500 cm³ or less than about 100 cm³ (as determined by sealing any openings on the external surfaces of the imaging device and measuring the volume of liquid displaced by the sealed imaging device). In some embodiments, the imaging device is configured such that the entire device may be arranged to fit into a pocket. For example, the ultrasound imaging device can be configured such that the entire device fits into a side pants pocket, according to certain embodiments.

In some such embodiments, the imaging device is portable such that it may be, for example, carried or otherwise manipulated by hand *(e.g.,* by a single hand). In some embodiments, the imaging device is completely self-contained with portable dimensions such that it can be manipulated with one hand.

In certain embodiments, the housing comprises a hand grip region *(e.g.,* a handle), which can be configured to be grasped by the user during operation of the imaging device. In some embodiments, the hand grip region can be between the ultrasound imaging unit and the display. For example, referring to FIG. 1, hand grip region 120 of imaging device 100 is positioned between imaging unit 104 and display 112. In certain embodiments, the hand grip region may be between the imaging interface of the ultrasound transducer and the display. The imaging interface of an ultrasound transducer is known to those of ordinary skill in the art to refer to the interface of the transducer from which the ultrasonic signal emanates. For example, referring to FIG. 1, ultrasound transducer 108 comprises imaging interface 109.

The hand grip region *(e.g.,* handle) may be positioned, in certain embodiments, such that it is directly above the imaging unit during operation of the imaging device. For example, referring to FIG. 1, hand grip region 120 is positioned such that it is directly above imaging unit 104 (as well as transducer 108 and imaging interface 109 of transducer 108) during operation of imaging device 100.

In some embodiments, the imaging device can be battery operated. For example, in certain embodiments, the imaging device is powered using a cell-phone class battery, such as a 2000 mAh Li-ion battery.

In certain embodiments the display can be integrated with the device housing *(e.g.,* such that display and the device housing form a monolithic unit). For example, referring to FIG. 1, device housing 102 is integrated with imaging unit 104 (and transducer 108) to form an integrated, monolithic unit. In addition, in FIG. 1, display 112 and device housing 102 are integrated to form a monolithic unit. In other embodiments the display can be detachable from the housing unit. In some such embodiments, the ultrasound imaging unit *(e.g.,* 104 in FIG. 1) can communicate with the display via wireless connection.

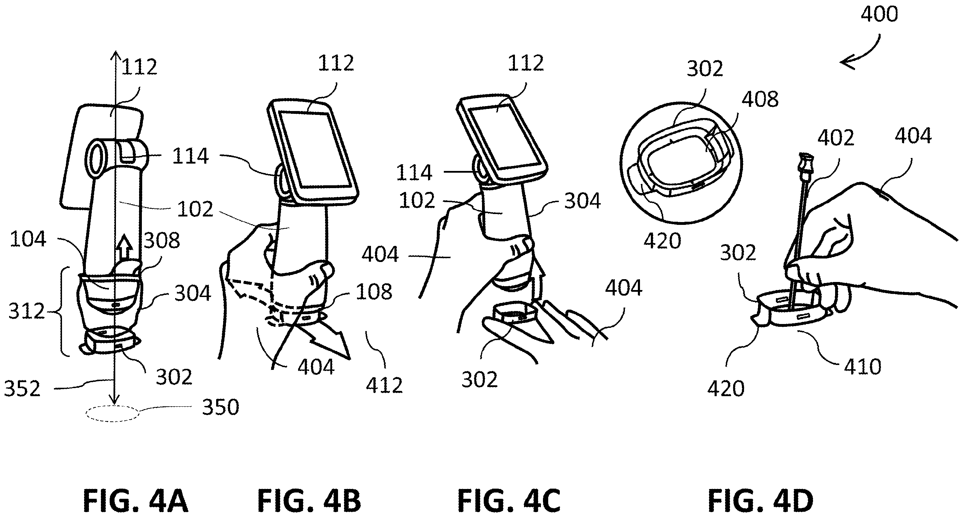

Certain of the imaging devices described herein can provide one or more of a variety of advantages, relative to prior devices. For example, according to certain embodiments, by attaching the display to the base of the housing (e.g., in direct contact with the base of the housing), the display can be located relatively close to the target site that is being imaged *(e.g.,* a subject such as a human subject). This can make, according to certain embodiments, operation of the imaging device easier than systems that include, for example, a transducer probe with a cable and the display that is not in the same vicinity as the user's hand and probe. In certain embodiments, the closest distance between the image display and the target site (e.g., skin surface 412 in FIGS. 4A-4D) is less than about 1 meter, less than about 500 cm, less than about 100 cm, less than about 50 cm, or less than about 25 cm.

Certain of the embodiments described herein do not include a separate transducer-to-display cable, which would otherwise complicate operation and handling of the imaging device. In addition, as noted above, the imaging device can be configured to be portable and adopt a form factor that can be more easily transported in a user's pocket. While other handheld ultrasound devices have been proposed with an attached screen (*i.e.,* no transducer-to-display cable), such systems possess a form factor where the hand is placed along a handle region that is extended from the transducer face. That is to say, in such systems, the longitudinal axis of the handle forms a smallest angle with the imaging plane of the imaging unit that is close to 90 degrees. In contrast, certain of the embodiments described herein place the hand immediately above the transducer face, which possesses the benefit of allowing for finer control of the scan plane. That is to say, certain of the embodiments described herein include a smallest angle between the longitudinal axis of the hand grip region and the imaging plane of the imaging unit that is relatively small *(e.g.,* a smallest angle of less than about 45°, less than about 30°, less than about 15°, or less than about 5°) and/or are configured such that the longitudinal axis of the hand grip region is substantially parallel to the imaging plane of the imaging unit. Those of ordinary skill in the art are familiar with the longitudinal axis of a hand grip region, which refers to the elongated axis around which the hand grasps when grasping the hand grip region. In certain embodiments, the longitudinal axis of the hand grip region corresponds to the longitudinal axis of the housing in which the hand grip region is formed.

According to certain embodiments, there may be a clinical benefit to using an ultrasound imaging device in which the display screen is housed within the same device housing as the imaging unit and/or is rotatable about the longitudinal axis of the device. In some embodiments, the rotating display can allow the user to adjust the display angle to account for different angles between the user's eye level and the central longitudinal axis of the device. Thus, the user may hold the imaging device above, below, to the left of, or to the right of their eye level and still have a good viewing angle to the display. The ability to adjust the eye level can be important for scanning different anatomies with the same device while still being able to view the display screen. As one example, performing a cardiac scan and a lumbar spine scan while maintaining the user's body in a substantially fixed position (e.g., standing or sitting) will generally necessitate positioning the imaging device such that the angle between the device and the user's eye level varies. Without a rotatable screen, the user would need to substantially move their body orientation in order to view the screen with sufficient clarity for both types of scans. With the rotatable screen, however, there is greater flexibility in uses of the ultrasound device, while maintaining a smaller form factor compared with ultrasound devices that have a separate transducer and display screen connected by a cable.

Moreover, positioning the hand grip region as illustrated in FIG. 1 (and other figures) can produce greater control over the position of the scan plane and/or can facilitate clinical acceptance. Precise control over the scan plane relative to the anatomy of interest is often important in ensuring that the anatomy is accurately captured by the scan plane and can be assessed via review of the display screen. In certain embodiments in which the hand grip region is configured such that the longitudinal axis of the hand grip region is substantially parallel to the imaging plane, the user's hand can be used to control the imaging device by placing the hand on the hand grip region while also resting the hand on the target skin surface. During testing of this configuration, it was discovered that arranging the imaging unit components in this manner leads to greater stability. Without wishing to be bound by any particular theory, it is believed that this increase in stability is observed because the hand, target skin surface, and device are all connected and can all move together. Frequently, a subject *(e.g.,* a patient) may move during an ultrasound scan *(e.g.,* due to discomfort, restlessness, or for another reason). Certain embodiments, including that illustrated in FIG. 1 and other figures can allow for a stable image to be captured regardless of patient motion.

In certain embodiments, the imaging device comprises an actuator on the hand grip region configured such that when the actuator is activated, data from the ultrasound imaging unit is recorded and/or manipulated. For example, in FIG. 1, imaging device 100 comprises actuator 130, which is in the form of a button. According to certain embodiments, when actuator 130 is depressed, data from the ultrasound imaging unit is recorded, for example, to memory within the ultrasound imaging device or to an external memory unit outside the imaging device *(e.g.,* after being transported via a wireless or wired connection between the imaging unit and the external memory unit). The data from the ultrasound imaging unit that is recorded can correspond to, for example, imaging data *(e.g.,* data related to a B-mode image, a C-mode image, an M-mode image, a tissue harmonic image, a three-dimensional image, a Color Doppler image, a Power Doppler image, a Pulse-wave Doppler image, a continuous wave Doppler image, an ultrasound contrast agent enhanced image, a B-flow image, or any other type of image). In this way, the actuator can be configured to take a "snapshot" of the data being collected by the ultrasound imaging device at that point in time during its use. In some embodiments, the actuator can be configured to perform one or more of a number of other functions including: video save, device power on/off, image settings adjustment (e.g., imaging mode, gain, frequency, contrast, depth), and/or menu navigation. In some embodiments, the imaging device 100 comprises a plurality of actuators, such as the actuator 130 in FIG. 1, which can perform different functions, such as the aforementioned functions.

While the actuator illustrated in FIG. 1 is a button, any suitable actuator type can be used. Examples include, but are not limited to, touch sensors (e.g., resistive, capacitive, optical), switches, proximity sensors, and/or optical sensors.

It can be advantageous, according to certain but not necessarily all embodiments, to locate the actuator on the hand grip region of the imaging device. Locating the actuator on the hand grip region can allow a user to activate the data saving function, or other aforementioned functions, relatively easily, for example, by simply activating the actuator using a digit of the hand (e.g., a finger and/or thumb) holding the imaging device. In some embodiments, the hand may activate the actuator for purposes of activating a desired function, such as a saving function, simultaneously while performing an image scan without requiring the user to reorient the hand on the hand grip region of the ultrasound device. That is to say, according to certain embodiments, the user does not need to reorient their hand to save an image during an ultrasound scan.

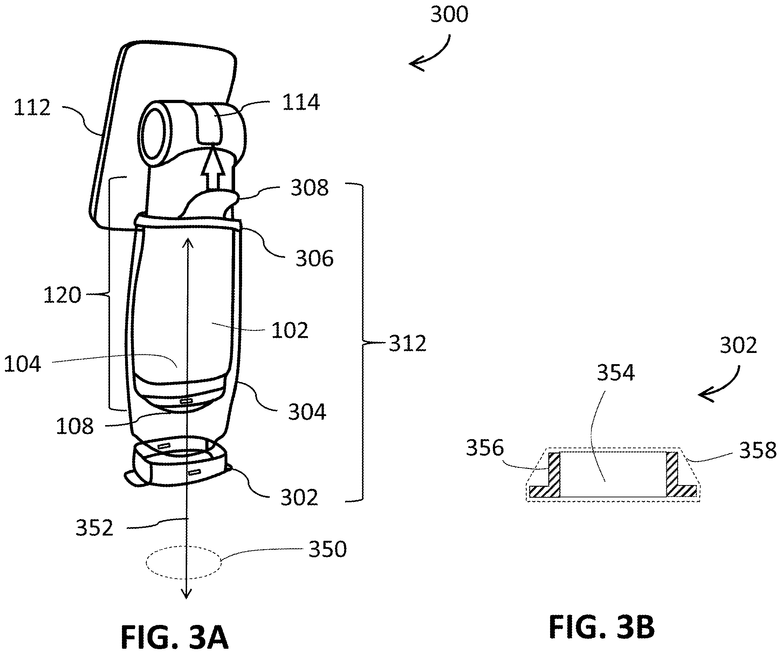

According to certain embodiments, the imaging device comprises a marking unit detachably coupled to the housing. For example, in FIG. 3A, imaging unit 300 comprises marking unit 302, which can be detachably coupled to housing 102. Similar arrangements are illustrated in the embodiments shown in FIGS. 4A-4D, 5A-5E, 6, and 7, described in more detail below.

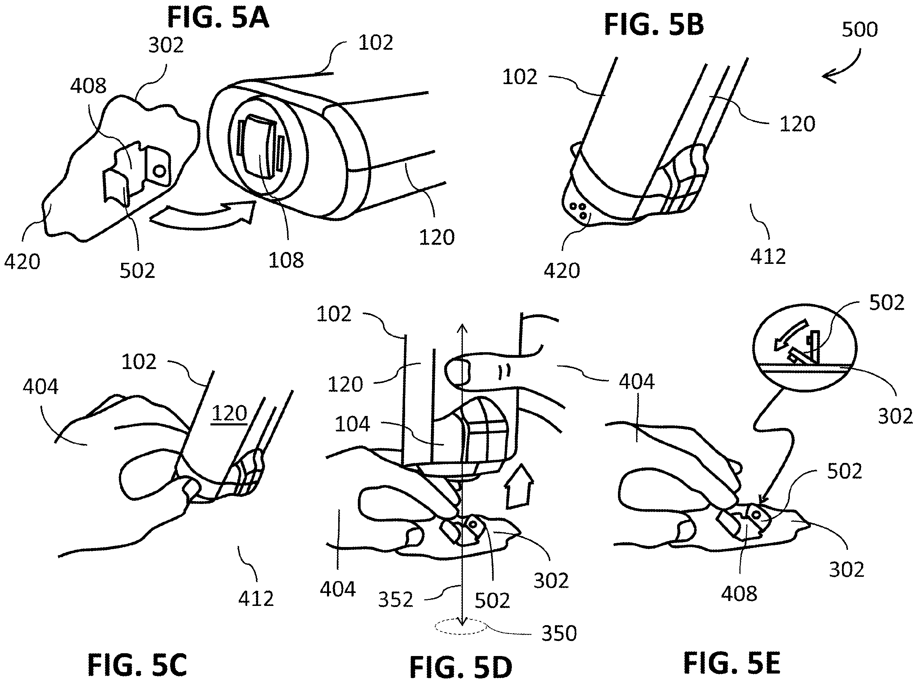

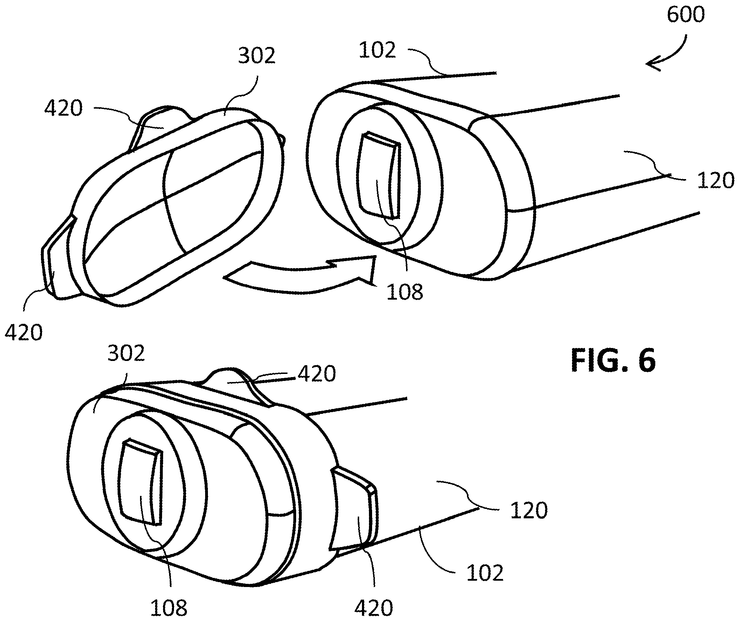

The marking unit and the housing can be detachably coupled in any suitable fashion. In some embodiments, the marking unit can be indirectly coupled to the housing. For example, as described in more detail below, according to certain embodiments, a cover body is attached to the housing. In some such embodiments, the marking unit is detachably coupled to the cover body. Exemplary embodiments of such an arrangement are illustrated in FIGS. 3A and 4A-4D. In certain embodiments, the marking unit is present alone, without the cover body. In some such embodiments, the marking unit is directly coupled to the housing. For example, in FIGS. 5A-5E and 6, marking unit 302 is directly coupled to the housing of the imaging unit. The marking unit can be "self-attaching" to the imaging device (or the cover body, when present), in certain embodiments. That is to say, in some embodiments, substantially no separate attaching material (such as a rubber band) is required to attach the marking unit to the imaging device (or to the cover body, when present). Examples of mechanisms that can be used to attach the marking unit to the imaging device (and/or the cover body, when employed) in this way include, but are not limited to, magnetic fittings *(e.g.,* a pair of magnets between the housing and the marking unit), mechanical attaching mechanisms (such as a fitted plastic inserts, spring-loaded retaining clips, frictional fittings, elastic bands, hook and loop fasteners, screw threads), and the like. Of course, other attachment mechanisms, such as adhesive-based attachment mechanisms, could also be used.

The marking unit can be configured to indicate proper placement of a probe (*e.g.,* a needle and/or a catheter) along a target that is to be imaged, in some embodiments. In certain embodiments, the marking unit is configured to identify a target surface location *(e.g.,* an insertion location) corresponding to a center of an imaging scan plane. The marking unit can comprise, in certain embodiments, a probe indicator configured to indicate proper placement of a probe at or near a target that is to be imaged. For example, in some embodiments, the marking unit comprises an identifying mark indicating the target surface location. The identifying mark can comprise, for example, a hole, an indentation, or other identifying mark.

In some such embodiments, the detachable marking unit can be mechanically removed from the imaging device during use. In some such embodiments, the detachable marking unit 302 can be left at or near the target site *(e.g.,* on the skin) to identify the center of the transducer along the skin after the transducer itself has been removed.

FIGS. 4A-4D are exemplary schematic illustrations outlining such use of the detachable marking unit. In FIG. 4A, the detachable marking unit is used to identify the location of needle insertion (i.e. the target insertion location 410) in a spinal anesthesia procedure, although use of the detachable marking unit to locate other types of target anatomy (e.g., blood vessels, nerves, joints, or organ tissues) is also possible. In FIG. 4A, marking unit 302 is placed over an imaging device hand grip region 120. Marking unit 302 can be configured to indicate proper placement of a needle (shown as 402 in FIG. 4D) along a target skin surface 412, which is to be the imaging surface, in some embodiments.

As shown in FIG. 4B, ultrasound imaging device 100 can be scanned across target skin surface 412 with a user's hand 404 until the user identifies the target anatomy in the ultrasound image displayed on device display 112. According to certain embodiments, marking unit 302 can be configured to identify a location along skin surface 412 that corresponds to the center of the scanning plane produced by ultrasound transducer 108 of the imaging device.

Next, as illustrated in FIG. 4C, marking unit 302 can be detached by user's hand 404 from both the device hand grip region 120 (and optional cover body 304, described in more detail below). In certain embodiments, the marking unit is configured to be attached to a target surface. For example, in some embodiments, the marking unit comprises an adhesive material configured to adhere the marking unit to a surface of a target *(e.g.,* the skin of a subject). In FIG. 4C, for example, marking unit 302 can comprise an adhesive material configured to adhere marking unit 302 to skin surface 412. Other attachment mechanisms could also be employed. For example, in some embodiments, the marking unit comprises a vacuum-generating unit configured to adhere the marking unit to a surface associated with the target. The marking unit 302 can comprise, for example, suction cups or other vacuum means to support adhesion of the marking unit to the target surface.

As noted above, in certain embodiments, the marking unit comprises, a probe indicator configured to indicate proper placement of a probe at or near a target that is to be imaged. For example, in some embodiments, the marking unit comprises an identifying mark indicating the target surface location. The identifying mark can comprise, for example, a hole, an indentation, or other identifying mark. In FIG. 4D, marking unit 302 comprises identifying marks 408, which are configured to indicate the proper placement of needle 402 along skin surface 412. In certain embodiments, the marking unit 302 comprises a hole 408 in the middle where the center of the transducer 108 was located when the unit 302 was detached from the imaging device housing, such that when left on skin 412, marking unit 302 identifies the place along skin surface 412 corresponding to the center of ultrasound scan plane 110.

According to certain embodiments, with the marking unit 302 adhered to the skin surface 412, the user can insert a probe 402 at the target insertion location 410 using the hole guide 408 along the marking unit 302.

The detachable marking unit, in some embodiments, can comprise a wing component. For example, referring to FIG. 4D, detachable marking unit 302 comprises wing 420. The wing component can be advantageous, according to certain although not necessarily all embodiments, because it can improve one's ability to adhere the marking unit to a target skin surface by increasing surface area of the marking unit along the target skin surface.

While identifying marks are illustrated in FIG. 4D as guiding the placement of the probe, other embodiments are also possible. For example, in some embodiments, the marking unit comprises a moveable tab configured to indicate a site corresponding to a center of a scan plane. The moveable tab can be configured, in certain embodiments, to make a visible indentation at a site corresponding to the center of the scan plane (which can correspond, for example, to the center of a cavity, hole, or other indicator formed in the marking unit). FIGS. 5A-5E is a set of schematic illustrations outlining the use of a moveable tab according to the invention to indicate a target region. In FIG. 5A, tab 502 can be folded into a center cavity 408 within the marking unit 302 to make an indentation along target skin surface 412 at target insertion location 410 corresponding to the center of cavity 408. In FIG. 5A ultrasound transducer 108 is illustrated with an exemplary embodiment of the marking unit 302 comprising a cavity 408 and moveable tabs 502. Marking unit 302 can be attached to the hand grip region 120 of the ultrasound device 100 to cover transducer region 302. FIG. 5B illustrates ultrasound device 100 with marking unit 302 attached and pressed against the target skin surface 412 where ultrasound imaging can be conducted. As shown in FIG. 5C, a user's hand 404 can press against marking unit tab regions 420 in order to remove marking unit 302 from ultrasound device 100 while maintaining marking unit 302 along the target skin surface 412 at the target insertion location 410. As illustrated in FIG. 5D, marking unit 302 can be removed from imaging device 100. As shown in FIG. 5E, the user's hand 404 can activate moveable tabs 502, which can make an indentation along target skin surface 412 at the target insertion location 410. According to certain embodiments, a probe may then be inserted at the point at which the indentation has been made.

FIG. 6 is a schematic illustration showing an exemplary mechanical attachment scheme that can be used to connect a marking unit 302 with an imaging device 100. The marking unit 302 in FIG. 6 is configured to "self-attach" to the device housing 102. Self-attachment can be achieved by previously described means including, in one embodiment, a form fitting between the device housing 102 and marking unit 302 such that frictional forces are sufficient to ensure attachment. The wings 420 of the marking unit 302 in FIG. 6 can be advantageous, according to certain although not necessarily all embodiments, because it can improve one's ability to adhere the marking unit to a target skin surface. Additionally, wing regions can provide areas whereby a user can press against to detach the marking unit 302 from the device housing 102.

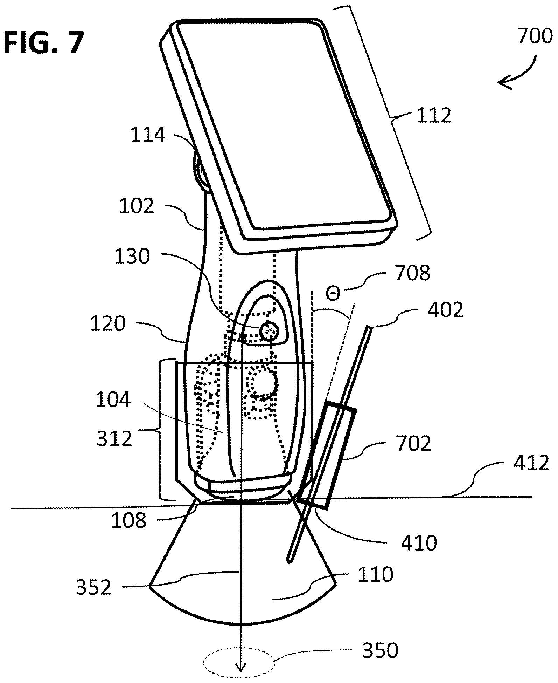

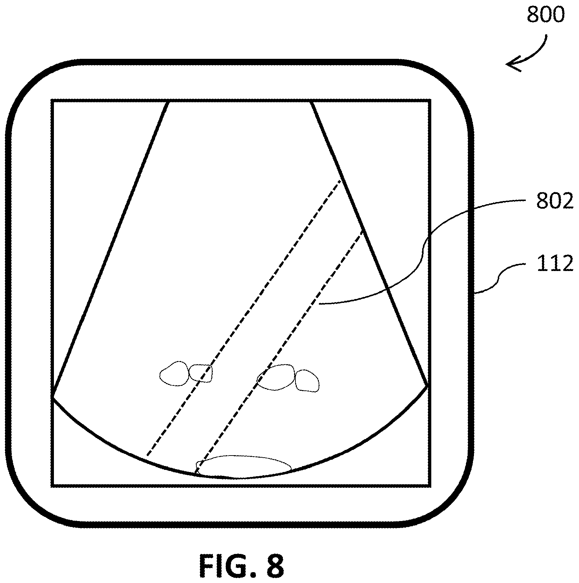

In some embodiments, the marking unit 302 comprises a sleeve 702 comprising an elongated lumen passing through it. The sleeve can be used as a probe indicator, as described above. For example, the elongated lumen of the sleeve can be used to guide or otherwise house an elongated device such as a probe 402. FIG. 7 is a schematic illustration of one set of embodiments in which a cover 312 comprises a sleeve 702, which is being used to house a probe, such as a needle 402. The sleeve 702 can be configured to control the angle 708 of the elongated probe 402 that is threaded through the lumen *(e.g.,* a needle 402 to be inserted at the target region 350). The imaging device display 112 can convey the angle 708 information with indicators such as, for example, dashed lines 802 superimposed on the ultrasound image. The user can then manually scan the imaging device 100 across the target skin surface 412 until the dashed line(s) 802 pass through the target of interest (e.g., blood vessel, nerve structure, epidural space) and then project the probe 402 through the sleeve 702. An example image display 112 in which overlaying indicator lines 802 are used to indicate the projected path of a probe 402 relative to the image if inserted through the sleeve 702 is illustrated in FIG. 8.

As noted above, certain aspects are related to configurations of a handheld ultrasound imaging device in which the target that is being imaged, the ultrasound imaging unit, and the marking unit are aligned during use. Alignment of these components during use can make location of target regions relatively easy for the user. According to certain embodiments, the marking unit is positioned such that, during use, a line extending between the target and the ultrasound imaging unit intersects the marking unit.

For example, referring to FIG. 3A, target region 350, marking unit 302, and imaging unit 104 are aligned such that line 352 (which extends infinitely, as indicated by the arrow at the ends of the line) intersects marking unit 302. It should be understood that a line is said to "intersect" a marking unit when the line passes through any region that lies within the outer geometric boundary of the marking unit, which includes both regions of the solid material that makes up the marking unit as well as voids (e.g., holes and the like) within such regions. As an illustrative example, FIG. 3B is a schematic cross-sectional schematic illustration of marking unit 302 shown in FIG. 3A. Marking unit 302 includes cavity 354 formed within a surrounding solid material 356, and defines outer geometric boundary 358. Although cavity 354 is not formed of any material per se, a line extending through cavity 354 would still be said to "intersect" marking unit 302.

Referring to FIGS. 4A-4C, marking unit 302, imaging unit 104, and target region 350 are also aligned such that line 352 intersects marking unit 302. In FIG. 5D, marking unit 302, imaging unit 104, and target region 350 are aligned such that line 352 intersects marking unit 302. In FIG. 7, imaging unit 104, and target region 350 are aligned such that line 352 intersects marking unit 302.

According to certain embodiments, the longitudinal axis of the housing intersects the marking unit (and, in some such embodiments, the longitudinal axis of the housing extends between the target and the ultrasound imaging unit). In some embodiments, the longitudinal axis of the hand grip region intersects the marking unit (and, in some such embodiments, the longitudinal axis of the hand grip region extends between the target and the ultrasound imaging unit).

According to certain embodiments, during use, the marking unit is positioned between the ultrasound imaging unit and the target that is to be imaged. For example, in FIG. 3A, marking unit 302 is positioned such that imaging unit 104 is above marking unit 302, and target region 350 is below marking unit 302. In other embodiments, the imaging unit could be located between the marking unit and the target region, for example, by sliding the marking unit up the hand grip region 120 of the housing.

In certain embodiments, a cover for an ultrasound imaging device is described. The cover comprises, in certain embodiments, a cover body configured to be attached to an imaging device, and a marking unit attached to the cover body and configured to indicate proper placement of a probe *(e.g.,* a needle and/or a catheter) along a target that is to be imaged. Such imaging can allow a target anatomy to be reached with the probe, according to certain embodiments.

FIG. 3A is an exemplary schematic illustration of a cover 312 including a cover body 304 and detachable marking unit 302 connected to an imaging device 100. In FIG. 3A, the cover body covers the hand grip region 120 *(e.g.,* including the imaging unit 104 and ultrasound transducer 108). The cover 312 can be used, in certain embodiments, as a sterile shield to the target skin surface 412 *(e.g.,* the skin of a subject). According to certain embodiments, cover 312 can comprise an elastomeric band 306 and tab 308, for example, to facilitate the placement of cover 312 over hand grip region 120.

The cover body can be "self-attaching" to the imaging device, in certain embodiments. That is to say, in some embodiments, substantially no separate attaching material (such as a rubber band) is required to attach the cover body to the imaging device. Examples of mechanisms that can be used to attach the cover body to the imaging device in this way include, but are not limited to, a pair of magnets between the cover body and the hand grip region, mechanical attaching mechanisms (such as a fitted plastic inserts, spring-loaded retaining clips, elastic bands), and the like.

In certain embodiments, cover body 304 and/or marking unit 302 may be disposable. In some embodiments, cover body 304 and/or marking unit 302 may be sterile.

In certain embodiments in which a cover body is employed, the cover body and the marking unit can be removably attached to each other. For example, the cover body and the marking unit can be attached to each other, in certain embodiments, such that separation of the cover body and the marking unit does not damage either of the cover body and the marking unit. In some embodiments, the cover body and the marking unit can be attached to each other such that separation of the cover body and the marking unit can be achieved by hand, and does not require an additional tool *(e.g.,* a screwdriver or other such tool). Examples of mechanisms that can be used to attach the marking unit to the imaging device (and/or the cover body, when employed) in this way include, but are not limited to, magnetic fittings *(e.g.,* a pair of magnets between the housing and the marking unit), mechanical attaching mechanisms (such as a fitted plastic inserts, spring-loaded retaining clips, frictional fittings, elastic bands, hook and loop fasteners, screw threads), and the like.

In some embodiments, the marking unit is configured such that when it is attached to the imaging device, an image can be produced without the use of transmission medium. According to certain embodiments, when marking unit is attached to the imaging device, an image can be produced without substantial acoustic attenuation derived from the marking unit and without the use of transmission medium. For example, in some embodiments, less than 6 dB round-trip acoustic attenuation is observed at 1 MHz center frequency, without the use of transmission medium, when the marking unit is positioned as described elsewhere herein (e.g., when the marking unit is aligned with the ultrasound imaging unit and the target, as described elsewhere herein). In some embodiments, the cover can be configured such that when it is attached to an ultrasound imaging device, received ultrasound data can be recorded for display of an image to the device display without the use of ultrasound gel or other ultrasound transmission medium being placed in between the transducer and the cover and/or marking unit. In contrast, other ultrasound imaging systems generally require the use of ultrasound gel to produce an image with acceptable image quality. The requirement for ultrasound transmission medium between the transducer and cover and/or marking unit can be eliminated, according to certain embodiments, by attaching the cover and/or marking unit to the transducer such that substantially no air is present between the two objects. For example, in some embodiments, an elastomeric band or other attaching mechanism can be configured such that the when the cover is attached to the ultrasound imaging device, it is pressed with high tension against the transducer surface. The tension can be made to be sufficiently high such that there substantially no air can be present between the two objects. In this way, the ultrasound beam can transmit between the two materials (*i.e.* transducer and cover) without requiring transmission medium. Exemplary materials from which the marking unit can be made include, for example, polyurethane, polyethylene, and silicone.

In some embodiments in which a cover body is employed (in addition to the marking unit), the cover is configured such that when it is attached to the imaging device, an image can be produced without substantial acoustic attenuation derived from the cover and without the use of transmission medium. For example, the cover can be configured, in certain embodiments, such that when it is attached to an ultrasound imaging device, received ultrasound data can be recorded for display of an image to the device display without the use of ultrasound gel or other ultrasound transmission medium being placed in between the transducer and the cover. The cover can be configured for us without transmission medium, for example, by using a cover material that is thin (e.g., less than about 5 mm thick) and/or by employing a self-attaching mechanism that holds the cover material against the transducer face with sufficient strength during operation of the imaging device. In this way, substantially no air is trapped between the cover and transducer face, and the cover itself is too thin to cause appreciable attenuation or reflections that can lead to unacceptable image quality (e.g., less than 6 dB round-trip acoustic attenuation at 1 MHz center frequency). Example cover materials can include, for example, polyurethane, polyethylene, and silicone.

As noted above, the ultrasound imaging devices described herein can produce ultrasound images using a variety of known techniques. In certain embodiments the ultrasound transducer (e.g., ultrasound transducer 108) can include a mechanically scanned single element transducer. In some embodiments, the ultrasound transducer can be a linear array, a two-dimensional array, or an annual array. In certain embodiments the ultrasound imaging unit (e.g., ultrasound imaging unit 104) can be configured to produce, for example, a B-mode image, a C-mode image, an M-mode image, a tissue harmonic image, a three-dimensional image, a Color Doppler image, a Power Doppler image, a Pulse-wave Doppler image, a continuous wave Doppler image, an ultrasound contrast agent enhanced image, a B-flow image, or any other mode or combination of modes whereby an image is formed from information received by the ultrasound transducer. Those of ordinary skill in the art of ultrasound understand that an ultrasound imaging unit generally comprises a combination of one or more of an ultrasound transducer, and circuitry and processing units for conditioning, processing, and transferring image data to the display unit. The ultrasound imaging unit can be contained within the device housing. For example, in some embodiments, the ultrasound imaging unit can comprise an ultrasound transducer, an ultrasonic signal conditioning circuit, and a processor circuit, which can be communicatively connected via a bus. The ultrasonic signal conditioning circuit can include a number of conventional processing circuitries such as beam-forming circuitry or other processing circuitry. For example, the ultrasonic signal conditioning circuit can be configured to amplify, phase-shift, time-gate, filter, or otherwise condition received ultrasonic information (e.g., echo information), such as provided to the processor circuit. In a further example, the receive path from each transducer element can include one or more of a low noise amplifier, a main-stage amplifier, a band-pass or a low-pass filter, or an analog-to-digital converter. In one example, one or more signal conditioning steps can be performed digitally, such as by using the processor circuit. The term processor is used to generically refer to digital circuitry that can be used to manipulate ultrasound information obtained from the ultrasound transducer. Such circuitry can include one or more of a field-programmable gate array (FPGA) or other programmable logic devices (PLDs), a microprocessor, a system-on-chip including one or more execution cores or other circuitry, a microcontroller, or one or more or other circuits. Those of ordinary skill in the art of ultrasound and image processing will understand that the signal conditioning and processing steps and their order of operation to be performed by the signal conditional circuit and processor circuit will vary depending on the desired image to be rendered to the display (e.g., B-mode image, C-mode image, M-mode image, tissue harmonic image, three-dimensional image, Color Doppler image, Power Doppler image, Pulse-wave Doppler image, continuous wave Doppler image, ultrasound contrast agent enhanced image, or B-flow image).

While several embodiments of the present invention have been described and illustrated herein, those of ordinary skill in the art will readily envision a variety of other means and/or structures for performing the functions and/or obtaining the results and/or one or more of the advantages described herein. The invention is defined in the claims. Those skilled in the art will readily appreciate that all parameters, dimensions, materials, and configurations described herein are meant to be exemplary and that the actual parameters, dimensions, materials, and/or configurations will depend upon the specific application or applications for which the teachings of the present invention is/are used.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Other elements may optionally be present other than the elements specifically identified by the "and/or" clause, whether related or unrelated to those elements specifically identified unless clearly indicated to the contrary. Thus, as a non-limiting example, a reference to "A and/or B," when used in conjunction with open-ended language such as "comprising" can refer, in one embodiment, to A without B (optionally including elements other than B); in another embodiment, to B without A (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of." "Consisting essentially of," when used in the claims, shall have its ordinary meaning as used in the field of patent law.

In the claims, as well as in the specification above, all transitional phrases such as "comprising," "including," "carrying," "having," "containing," "involving," "holding," and the like are to be understood to be open-ended, i.e., to mean including but not limited to. Only the transitional phrases "consisting of' and "consisting essentially of' shall be closed or semi-closed transitional phrases, respectively.

## Claims

1. A handheld ultrasound imaging device (100), comprising:
a housing (102) comprising:
an ultrasound imaging unit (104);
a hand grip region (120) between the ultrasound imaging unit and a rotatable display; and the rotatable display (112) connected to the housing via a pivot (114), wherein the pivot (114) allows for rotation of the display (112), relative to the hand grip region (120), about at least two rotational axes by at least about 30 degrees, and
a marking unit (302) detachably coupled to the housing (102) and comprising a probe indicator configured to indicate proper placement of a probe (402) at or near a target (350) that is to be imaged, wherein, during use, the marking unit (302) is arranged to be positioned between the ultrasound imaging unit (104) and the target (350) that is to be imaged, such that, during use, a line extending between the target (350) and the ultrasound imaging unit (104) intersects the marking unit (302),
wherein the marking unit (302) comprises a moveable tab (502) configured to indicate a site corresponding to a center of a scan plane (110),
wherein the moveable tab (502) is foldable into a center cavity (408) within the marking unit (302) to make a visible indentation at a site corresponding to the center of the scan plane (110) when the housing (102) has been detached from the marking unit (302).

2. The imaging device of claim 1, wherein the marking unit (302) comprises an adhesive material configured to adhere the marking unit (302) to a surface (412) associated with the target.

3. The imaging device of any one of claims 1 - 2, wherein the housing (102) is elongated and comprises a longitudinal axis (106).

4. The imaging device of claim 3, wherein a smallest angle between a scanning plane (110) of the ultrasound imaging unit (104) and the longitudinal axis (106) of the housing (102) is less than 45 degrees.

5. The imaging device of claim 3, wherein the scanning plane (110) of the ultrasound imaging unit (104) is substantially parallel to the longitudinal axis (106) of the housing (102).

6. The imaging device of any one of claims 1 - 5, wherein the pivot (114) comprises a ball-and-socket arrangement and the rotatable display (112) is configured to be rotated such that the display can be oriented in a first position substantially parallel to a scanning plane (110) of the imaging unit (104) and in a second position substantially perpendicular to the scanning plane (110) of the imaging unit (104).

7. The imaging device of any of the preceding claims, comprising a cover body (304) attached to the housing.

8. The imaging device of claim 7, wherein said cover body (304) comprises polyurethane, polyethylene, or silicone.

## Patentansprüche

1. Handgehaltenes Ultraschallbildgebungsgerät (100), das Folgendes umfasst:
ein Gehäuse (102), das Folgendes umfasst:
eine Ultraschallbildgebungseinheit (104);
einen Handgriffbereich (120) zwischen der Ultraschallbildgebungseinheit und einem drehbaren Display; wobei
das drehbare Display (112) über ein Drehgelenk (114) mit dem Gehäuse verbunden ist, wobei das Drehgelenk (114) eine Drehung des Displays (112) relativ zum Handgriffbereich (120) um mindestens zwei Drehachsen um mindestens etwa 30 Grad ermöglicht, und
eine Markierungseinheit (302), die abnehmbar mit dem Gehäuse (102) gekoppelt ist und einen Sondenindikator umfasst, der zum Anzeigen der richtigen Platzierung einer Sonde (402) an oder nahe einem abzubildenden Ziel (350) konfiguriert ist, wobei die Markierungseinheit (302) zum Positionieren, beim Gebrauch, zwischen der Ultraschallbildgebungseinheit (104) und dem abzubildenden Ziel (350) ausgelegt ist, so dass beim Gebrauch eine sich zwischen dem Ziel (350) und der Ultraschallbildgebungseinheit (104) erstreckende Linie die Markierungseinheit (302) schneidet,
wobei die Markierungseinheit (302) eine bewegliche Lasche (502) umfasst, die zum Anzeigen einer Stelle entsprechend einer Mitte einer Abtastebene (110) konfiguriert ist,
wobei die bewegliche Lasche (502) in einen zentralen Hohlraum (408) innerhalb der Markierungseinheit (302) faltbar ist, um eine sichtbare Vertiefung an einer Stelle entsprechend der Mitte der Abtastebene (110) zu bilden, wenn das Gehäuse (102) von der Markierungseinheit (302) abgenommen wurde.

2. Bildgebungsgerät nach Anspruch 1, wobei die Markierungseinheit (302) ein Haftmaterial umfasst, das zum Kleben der Markierungseinheit (302) an eine mit dem Ziel assoziierte Oberfläche (412) konfiguriert ist.

3. Bildgebungsgerät nach einem der Ansprüche 1-2, wobei das Gehäuse (102) länglich ist und eine Längsachse (106) aufweist.

4. Bildgebungsgerät nach Anspruch 2, bei dem ein kleinster Winkel zwischen einer Abtastebene (110) der Ultraschallbildgebungseinheit (104) und der Längsachse (106) des Gehäuses (102) kleiner als 45 Grad ist.

5. Bildgebungsgerät nach Anspruch 3, bei dem die Abtastebene (110) der Ultraschallbildgebungseinheit (104) im Wesentlichen parallel zur Längsachse (106) des Gehäuses (102) verläuft.

6. Bildgebungsgerät nach einem der Ansprüche 1-5, wobei das Drehgelenk (114) eine Kugelgelenkanordnung umfasst und das drehbare Display (112) so konfiguriert ist, dass es gedreht werden kann, so dass das Display in einer ersten Position im Wesentlichen parallel zu einer Abtastebene (110) der Bildgebungseinheit (104) und in einer zweiten Position im Wesentlichen lotrecht zur Abtastebene (110) der Bildgebungseinheit (104) orientiert werden kann.

7. Bildgebungsgerät nach einem der vorherigen Ansprüche, das einen am Gehäuse angebrachten Abdeckkörper (304) umfasst.

8. Bildgebungsgerät nach Anspruch 7, wobei der genannte Abdeckkörper (304) Polyurethan, Polyethylen oder Silikon umfasst.

## Revendications

1. Dispositif d'imagerie ultrasonore portatif (100), comprenant :
un boîtier (102) comprenant :
une unité d'imagerie ultrasonore (104) ;
une région formant poignée (120) entre l'unité d'imager ultrasonore et un écran rotatif ; et
l'écran rotatif (112) connecté au boîtier par l'intermédiaire d'un pivot (114), le pivot (114) permettant une rotation de l'écran (112), par rapport à la région formant poignée (120), autour d'au moins deux axes de rotation, d'au moins environ 30 degrés ; et
une unité de marquage (302) accouplée amovible au boîtier (102) et comprenant un indicateur de sonde configuré pour indiquer un emplacement approprié d'une sonde (402) au niveau d'une cible (350) à imager ou à proximité de celle-ci, lors de l'utilisation l'unité de marquage (302) étant conçue pour être positionnée entre l'unité d'imagerie ultrasonore (104) et la cible (350) à imager, de sorte que, lors de l'utilisation, une ligne s'étendant entre la cible (350) et l'unité d'imagerie ultrasonore (104) croise l'unité de marquage (302),
l'unité de marquage (302) comprenant une patte mobile (502) configurée pour indiquer un site correspondant à un centre d'un plan de balayage (110),
la patte mobile (502) étant pliable dans une cavité centrale (408) à l'intérieur de l'unité de marquage (302) pour produire un retrait visible au niveau d'un site correspondant au centre du plan de balayage (110) quand le boîtier (102) a été détaché de l'unité de marquage (302).

2. Dispositif d'imagerie selon la revendication 1, dans lequel l'unité de marquage (302) comprend un matériau adhésif configuré pour que l'unité de marquage (302) adhère à une surface (412) associée à la cible.

3. Dispositif d'imagerie selon l'une quelconque des revendications 1 et 2, dans lequel le boîtier (102) est allongé et comprend un axe longitudinal (106).

4. Dispositif d'imagerie selon la revendication 3, dans lequel un angle minimum entre un plan de balayage (110) de l'unité d'imagerie ultrasonore (104) et l'axe longitudinal (106) du boîtier (102) est inférieur à 45 degrés.

5. Dispositif d'imagerie selon la revendication 3, dans lequel le plan de balayage (110) de l'unité d'imagerie ultrasonore (104) est sensiblement parallèle à l'axe longitudinal (106) du boîtier (102).

6. Dispositif d'imagerie selon l'une quelconque des revendications 1 à 5, dans lequel le pivot (114) comprend un agencement sphéroïde et l'écran rotatif (112) est configuré pour être tourné de sorte que l'écran puisse être orienté dans une première position sensiblement parallèle à un plan de balayage (110) de l'unité d'imagerie (104) et dans une seconde position sensiblement perpendiculaire au plan de balayage (110) de l'unité d'imagerie (104).

7. Dispositif d'imagerie selon l'une quelconque des revendications précédentes, comprenant un corps formant couvercle (304) fixé au boîtier.

8. Dispositif selon la revendication 7, dans lequel ledit corps formant couvercle (304) comprend du polyuréthane, du polyéthylène ou de la silicone.
